(19) **Europäisches Patentamt**
**European Patent Office**
**Office européen des brevets**

(11) **EP 2 585 091 B1**

(12) **EUROPEAN PATENT SPECIFICATION**

(45) Date of publication and mention
of the grant of the patent:
**10.09.2014 Bulletin 2014/37**

(51) Int Cl.:
*A61K 38/08* (2006.01)  *A61K 47/34* (2006.01)
*A61P 35/00* (2006.01)  *A61K 9/52* (2006.01)
*C08G 63/08* (2006.01)

(21) Application number: **11731522.6**

(22) Date of filing: **23.06.2011**

(86) International application number:
**PCT/JP2011/064992**

(87) International publication number:
**WO 2011/162413 (29.12.2011 Gazette 2011/52)**

(54) **SUSTAINED-RELEASE FORMULATION COMPRISING A METASTIN DERIVATIVE**

FORMULIERUNG MIT VERZÖGERTER FREISETZUNG ENTHALTEND EIN METASTINDERIVAT

FORMULE À LIBÉRATION PROLONGÉE COMPRENANT UN DÉRIVÉ DE METASTIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **25.06.2010 JP 2010144792**

(43) Date of publication of application:
**01.05.2013 Bulletin 2013/18**

(73) Proprietor: **Takeda Pharmaceutical Company
Limited
Osaka-shi, Osaka 541-0045 (JP)**

(72) Inventors:
• **FUTO, Tomomichi**
**Osaka-shi**
**Osaka 532-0024 (JP)**
• **TAIRA, Hikaru**
**Osaka-shi**
**Osaka 532-0024 (JP)**
• **MIZUKAMI, Seitaro**
**Osaka-shi**
**Osaka 532-0024 (JP)**
• **MURATA, Naoyuki**
**Osaka-shi**
**Osaka 532-0024 (JP)**

(74) Representative: **Kingsbury, Oliver William
Elkington and Fife LLP
Prospect House
8 Pembroke Road
Sevenoaks, Kent TN13 1XR (GB)**

(56) References cited:
**EP-A1- 1 310 517       WO-A1-2007/072997
WO-A1-2008/050897**

• **DATABASE WPI Week 200309 Thomson
Scientific, London, GB; AN 2003-103386
XP000002659403, & WO 02/085399 A1 (TAKEDA
CHEM IND LTD) 31 October 2002 (2002-10-31)**

SEQUENCE LISTING

<110> Takeda Pharmaceutical Company Limited

<120> Sustained-release formulation

<130> PCT11-0023

<150> JP 2010-144792
<151> 2010-06-25

<160> 2

<170> PatentIn version 3.5

<210> 1
<211> 9
<212> PRT
<213> Artificial Sequence

<220>
<223> Synthetic peptide

<220>
<221> MISC_FEATURE
<222> (1).. (1)
<223> Xaa is Acetyl-D-Tyrosine.

<220>
<221> MISC_FEATURE
<222> (2).. (2)
<223> Xaa is trans-4-hydroxyproline.

<220>
<221> MISC_FEATURE
<222> (6).. (6)
<223> Xaa is Azaglycine.

<220>
<221> MISC_FEATURE
<222> (8)..(8)
<223> Xaa is N-omega-methylarginine.

<400> 1

```
Xaa Xaa Asn Thr Phe Xaa Leu Xaa Trp

       1                       5
```

<210> 2
<211> 9
<212> PRT
<213> Artificial Sequence

<220>
<223> Synthetic peptide

<220>
<221> MISC_FEATURE
<222> (1).. (1)
<223> Xaa is Acetyl-D-tyrosine.

<220>
<221> MISC_FEATURE
<222> (2).. (2)
<223> Xaa is D-tryptophan.

<220>
<221> MISC_FEATURE
<222> (6).. (6)
<223> Xaa is Azaglycine.

<220>
<221> MISC_FEATURE
<222> (8).. (8)
<223> Xaa is N-omega-methylarginine.

<400> 2

```
Xaa Xaa Asn Thr Phe Xaa Leu Xaa Trp
 1                   5
```

**Description**

## 1. TECHNICAL FIELD

The present invention relates to a novel sustained-release formulation and the like which can effectively treat cancer and the like.

## 2. BACKGROUND OF THE INVENTION

As a stable metastin derivative having an excellent metastin-like activity, for example, a compound described in Patent Literature 1 is known. Furthermore, as a sustained-release formulation containing metastin or a derivative thereof, for example, the formulation described in Patent Literature 2 is known.

[Citation List]

[Patent Literature]

[Patent Literature 1] WO2007/72997
[Patent Literature 2] WO02/85399

## 3. SUMMARY OF THE INVENTION

[Problem to be solved by the Invention]

To obtain benefits from reduction of side effects by requiring no high dosage amount in order to obtain medicinal effects, an improvement of patient's convenience and overcoming pain by reduction of frequency of administration as well as producing medicinal effects over a long period of time, it is desired to develop a sustained-release formulation being capable of slowly releasing a metastin derivative over a long period of time and having excellent properties as a clinical medicine. It is particularly desired to develop a formulation which gives stable and sustained release of compound (I) or a salt thereof over a long period of time.

[Solution to the Problem]

The present inventors conducted intensive studies with a view to solving the aforementioned problems, and as a result, found that a sustained-release formulation which contains a metastin derivative or a salt thereof and a lactic acid polymer having a weight average molecular weight of about 5,000 to about 40,000 or a salt thereof according to the present application has excellent properties required for a clinical medicine in medicinal effects, safety, stability, dosage amount, dosage form and usage, and finally achieved the present invention.
More specifically, the present invention relates to the following sustained-release formulation and a method for producing the same.

[1] A sustained-release formulation comprising a compound represented by Formula:

$$\text{Ac-D-Tyr-Hyp-Asn-Thr-Phe-AzaGly-Leu-Arg(Me)-Trp-NH}_2 \qquad \text{(I)}$$

(in the specification, sometimes simply referred to as compound (I)) or a salt thereof, and a lactic acid polymer having a weight average molecular weight of about 5,000 to about 40,000 or a salt thereof;

[2] The sustained-release formulation according to claim [1] above, wherein the weight average molecular weight of the lactic acid polymer or a salt thereof is about 13,000 to about 17,000;

[3] The sustained-release formulation according to claim [1], which is a 3 to 6-month sustained-release formulation;

[4] The sustained-release formulation according to claim [1] above, which is a therapeutic or prophylactic agent for cancer;

[5] The sustained-release formulation according to claim [1] above, which is a parenteral agent; and

[6] A sustained-release formulation according to claim [1] for use in treating or preventing cancer.

[7] A method for producing the sustained-release formulation according to claim [1] above, comprising subjecting

(1) a W/O/W emulsion obtained by emulsifying a W/O emulsion composed of an internal water phase containing compound (I) or a salt thereof, and an oil phase containing the lactic acid polymer or a salt thereof, or

(2) an O/W emulsion obtained by emulsifying an oil phase containing compound (I) or a salt thereof and the lactic acid polymer or a salt thereof;

to an in-water drying method;

[8] The method according to claim [7], comprising subjecting an O/W emulsion obtained by emulsifying an oil phase containing compound (I) or a salt thereof and the lactic acid polymer or a salt thereof, to an in-water drying method.

Furthermore, the sustained-release formulation of the present invention may be used in a therapeutic method according to the following aspects.

[9] A sustained-release formulation comprising compound (I) or a salt thereof and a lactic acid polymer having a weight average molecular weight of about 5,000 to about 40,000 or a salt thereof, which is used such that compound (I) or a salt thereof is administered to a patient in a dose of about 0.01 to about 4 mg/kg body weight at intervals of once 3 weeks or more (preferably, 1 month);

[10] A sustained-release formulation comprising compound (I) or a salt thereof and a lactic acid polymer having a weight average molecular weight of about 5,000 to about 40,000 or a salt thereof, which is used such that compound (I) or a salt thereof is administered to a patient in a dose of about 0.03 to about 12 mg/kg body weight at intervals of once 2 months or more (preferably, 3 months);

[11] A sustained-release formulation comprising compound (I) or a salt thereof and a lactic acid polymer having a weight average molecular weight of about 5,000 to about 40,000 or a salt thereof, which is used such that compound (I) or a salt thereof is administered to a patient in a dose of about 0.06 to about 24 mg/kg body weight at intervals of once 4 months or more (preferably, 6 months);

[12] The sustained-release formulation according to any of items [9] to [11] above produced by a method using (1) a W/O/W emulsion or (2) an O/W emulsion;

[13] The sustained-release formulation according to any of items [1] to [5] and [9] to [12] above for use in treating or preventing cancer (for example, lung cancer, stomach cancer, liver cancer, pancreatic cancer, large bowel cancer, rectal cancer, colon cancer, prostatic cancer, ovary cancer, uterine cervix cancer, breast cancer, kidney cancer, bladder cancer, brain tumor), a pancreatic disease (for example, acute or chronic pancreatitis, pancreatic cancer), chorioma, hydatidiform mole, invasive mole, miscarriage, fetus hypogenesis, anomaly of saccharometabolism, lipidosis and abnormal childbirth;

[14] The sustained-release formulation according to item [13] above, which is a microcapsule formulation; and

[15] A method for treating or preventing cancer (for example, lung cancer, stomach cancer, liver cancer, pancreatic cancer, large bowel cancer, rectal cancer, colon cancer, prostatic cancer, ovary cancer, uterine cervix cancer, breast cancer, kidney cancer, bladder cancer, brain tumor), a pancreatic disease (for example, acute or chronic pancreatitis), chorioma, hydatidiform mole, invasive mole, miscarriage, fetus hypogenesis, anomaly of saccharometabolism, lipidosis and abnormal childbirth, comprising: administering an effective amount of the sustained-release formulation according to item [13] or [14] above to a mammal.

[Advantageous Effects of Invention]

The sustained-release formulation of the present invention slowly and stably releases compound (I) or a salt thereof over a long period of time (e.g., 3 weeks or more) and also exerts medicinal effects of compound (I) or a salt thereof over a long period of time. Furthermore, the sustained-release formulation of the present invention provides improved patient's convenience by reducing frequency of administration, and is an excellent formulation as a clinical medicine.

**4. MODE FOR CARRYING OUT THE INVENTION**

The present invention will be more specifically described below.

The present invention is defined by the claims.

The present invention provides a sustained-release formulation containing a metastin derivative (in the specification, sometimes simply referred to as compound (I)) represented by the following formula:

Ac-D-Tyr-Hyp-Asn-Thr-Phe-AzaGly-Leu-Arg(Me)-Trp-NH$_2$     (I)

(SEQ ID NO: 1)

or a salt thereof

Compound (I) to be used in the present invention may be present in the form of a salt. As the salt formed with compound (I), a pharmacologically acceptable salt is particularly preferable. Examples of such a salt include salts with inorganic acids (for example, hydrochloric acid, phosphoric acid, hydrobromic acid, sulfuric acid); salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid); salts with inorganic bases (for example, alkali metal salts such as a sodium salt and potassium salt; alkaline earth metal salts such as a calcium salt and a magnesium salt; an aluminium salt, an ammonium salt) and salts with organic bases (for example, trimethylamine, triethylamine, pyridine, picoline, ethanolamine, diethanolamine, triethanolamine, dicyclohexylamine, N,N-dibenzylethylenediamine).

Preferable examples of the salts formed with compound (I) that can be used in the present invention include salts with acetic acid.

Compound (I) or a salt thereof to be used in the present invention can be produced by a known peptide synthesis method, and more specifically, produced by the method described in WO2007/72997.

The sustained-release formulation of the present invention contains a lactic acid polymer having a weight average molecular weight of about 5,000 to about 40,000 (in the specification, sometimes simply referred to as a lactic acid polymer) or a salt thereof in addition to compound (I) or a salt thereof

In the present invention, the lactic acid polymer refers to a polymer consisting of lactic acid alone.

The weight average molecular weight of the lactic acid polymer or a salt thereof to be used in the present invention is about 5,000 to about 40,000, preferably about 5,000 to about 30,000, further preferably about 6,000 to about 20,000 and further more preferably about 13,000 to about 17,000.

The polydispersity of the lactic acid polymer or a salt thereof (weight average molecular weight/number average molecular weight) is preferably about 1.2 to about 4.0 and further preferably about 1.5 to about 3.5.

The weight average molecular weight and the polydispersity used in the specification refer to values obtained by gel permeation chromatographic (GPC) measurement. The weight average molecular weight and the content of each polymer are polystyrene-equivalent weight average molecular weight, which is obtained by GPC measurement using, for example, a mono disperse polystyrene as a standard substance, and the content of each polymer calculated from this, respectively. The weight average molecular weight and the content of each polymer can be measured, for example, by a high-speed GPC apparatus (HLC-8120 GPC manufactured by Tosoh Corporation). As a column, Super H4000 x 2 and Super H2000 (each manufactured by Tosoh Corporation) can be used. As a mobile phase, tetrahydrofuran can be used and the flow rate can be set to 0.6 mL/min. As a detection method, a differential refractive index can be used.

Note that as the lactic acid polymer and a salt thereof, a commercially available product can be used.

In the present invention, the lactic acid polymer may be present in the form of a salt. Examples of the salt include salts with inorganic bases (e.g., an alkali metal such as sodium and potassium, an alkaline earth metal such as calcium and magnesium) and organic bases (e.g., an organic amine such as triethylamine, a basic amino acid such as arginine) or salts with transition metals (e.g., zinc, iron, copper) and complex salts.

The sustained-release formulation of the present invention is, for example, produced by mixing compound (I) or a salt thereof and a lactic acid polymer or a salt thereof, and, if necessary, molding thus-obtained mixture. The amount of compound (I) or a salt thereof to be used is, for example, about 0.01 to about 50% (w/w) relative to a lactic acid polymer or a salt thereof, and preferably about 0.1 to about 30% (w/w).

Now, the method for producing a sustained-release formulation according to the present invention will be more specifically described below.

    (1) Method for producing rod-form molding and the like

(1-a)

A lactic acid polymer or a salt thereof is dissolved in an organic solvent (preferably dichloromethane, etc.) and an aqueous solution of compound (I) or a salt thereof is added and then emulsified. The resulting emulsion is dried in a vacuum to obtain powder having compound (I) or a salt thereof and the lactic acid polymer or a salt thereof uniformly dispersed therein. The powder is warmed and cooled to obtain moldings of disk form, film form, rod form and the like. The warming temperature is, for example, about 50 to about 100°C and cooling temperature is, for example, about 0 to about 40°C. The amount of compound (I) or a salt thereof to be used varies depending upon the type of compound (I) or a salt thereof, desired pharmacological effect and duration of the effect, etc.; however, it is, for example, about 0.01 to about 50% (w/w) relative to the lactic acid polymer or a salt thereof, preferably about 0.1 to about 30% (w/w), and particularly preferably about 1 to about 20% (w/w).

(1-b)

A lactic acid polymer or a salt thereof is dissolved in an organic solvent (preferably dichloromethane, etc.) and compound (I) or a salt thereof is uniformly dispersed. The resulting dispersion is dried in a vacuum to obtain powder of the lactic acid polymer or a salt thereof in which compound (I) or a salt thereof is uniformly dispersed. The powder was warmed and cooled to obtain moldings of disk form, film form, rod form and the like. The warming temperature, cooling temperature and amount of compound (I) or a salt thereof to be used are the same as described in the above section (1-a).

(2) Method for producing microcapsule (also referred to as microsphere)

(2-a) In-water drying method

Microcapsules are produced by subjecting (i) a W (internal water phase)/O (oil phase)/W (external water phase) emulsion, which is obtained by emulsifying a W (internal water phase)/O (oil phase) emulsion composed of an internal water phase containing compound (I) or a salt thereof and an oil phase containing a lactic acid polymer or a salt thereof, or (ii) an O (oil phase)/W (external water phase) emulsion, which is obtained by emulsifying an oil phase containing compound (I) or a salt thereof and a lactic acid polymer or a salt thereof, to an in-water-drying method.

The above emulsion (i), that is, the W/O emulsion composed of an internal water phase containing compound (I) or a salt thereof and an oil phase containing a lactic acid polymer or a salt thereof, is produced as follows.

First, compound (I) or a salt thereof is dissolved, dispersed or suspended in water to produce the internal water phase. The concentration of compound (I) or a salt thereof in water is, for example, 0.001 to 90% (w/w) and preferably 0.01 to 80% (w/w).

The amount of compound (I) or a salt thereof to be used varies depending upon the type of compound (I) or a salt thereof, desired pharmacological effect, duration of the effect and the like; it is, for example, about 0.01 to about 50% (w/w) relative to a lactic acid polymer or a salt thereof, preferably about 0.1 to about 30% (w/w) and further preferably about 1 to about 20% (w/w).

If necessary, to enhance uptake of compound (I) or a salt thereof in a microcapsule, a drug retaining substance such as gelatin, agar, sodium alginate, polyvinyl alcohol or basic amino acid (for example, arginine, histidine, lysine), may be added to an internal water phase. The amount of the drug retaining substance to be added is usually about 0.01 to about 10 fold by weight relative to compound (I) or a salt thereof.

The internal water phase may be once lyophilized into powder and thereafter dissolved by adding water so as to obtain an appropriate concentration and then put in use.

Separately, a lactic acid polymer or a salt thereof is dissolved in an organic solvent to produce an oil phase.

Examples of the organic solvent include halogenated hydrocarbons (for example, dichloromethane, chloroform, chloroethane, trichloroethane, carbon tetrachloride), fatty acid esters (for example, ethyl acetate, butyl acetate) and aromatic hydrocarbons (for example, benzene, toluene, xylene). Among others, dichloromethane is preferable.

The concentration of a lactic acid polymer or a salt thereof in an organic solvent varies depending upon the type and weight average molecular weight of the lactic acid polymer or a salt thereof and the type of organic solvent; usually, a value expressed by the formula:

$$[\text{weight of a lactic acid polymer or a salt thereof} / (\text{weight of an organic solvent} + \text{weight of a lactic acid polymer or a salt thereof})] (\times 100\%)$$

is about 0.01 to about 90% (w/w) and preferably about 0.01 to about 70% (w/w).

The oil phase desirably contains no insoluble matter.

To the organic solvent solution (oil phase) of a lactic acid polymer or a salt thereof thus obtained, an aqueous solution, dispersion or suspension (internal water phase) of compound (I) or a salt thereof is added, dispersed and emulsified by a homomixer, etc., to produce a W/O emulsion.

When the W/O emulsion is produced at room temperature (about 19 to 25°C), the resulting W/O emulsion changes with the passage of time to a state (e.g., gelatinous state), which is unfavorable to secondary emulsification (later described). In this case, it is sometimes difficult to produce microcapsules in high yield (the yield used herein refers to a ratio of the weight of compound (I) or a salt thereof contained in microcapsules to the weight of compound (I) or a salt thereof used for a W/O emulsion).

To prevent such a change, it is preferred that the temperature of a W/O emulsion produced is controlled to be 31°C or more (preferably 31 to 33°C).

On the other hand, the above emulsion (ii), that is, the oil phase containing compound (I) or a salt thereof and a lactic acid polymer or a salt thereof, is produced as follows.

First, an organic solvent solution of a lactic acid polymer or a salt thereof is produced. As the organic solvent, the same organic solvent used for producing the above W/O emulsion is used.

The concentration of a lactic acid polymer or a salt thereof in an organic solvent solution varies depending upon the type and weight average molecular weight of the lactic acid polymer or a salt thereof and the type of organic solvent; usually, a value expressed by the formula:

$$[\text{weight of a lactic acid polymer or a salt thereof}/(\text{weight of an organic solvent} + \text{weight of a lactic acid polymer or a salt thereof})] \, (\times \, 100\%)$$

is about 0.01 to about 70% (w/w) and preferably about 1 to about 60% (w/w).

Next, compound (I) or a salt thereof is dissolved or suspended in the organic solvent solution of a lactic acid polymer or a salt thereof to prepare an oil phase. The oil phase can be produced also by dissolving or suspending a solution, which is prepared by dissolving compound (I) or a salt thereof in an alcohol, in the organic solvent solution of a lactic acid polymer or a salt thereof. Examples of the alcohol for dissolving compound (I) or a salt thereof include methanol.

The amount of compound (I) or a salt thereof to be used may be selected such that the ratio of compound (I) or a salt thereof relative to a lactic acid polymer or a salt thereof is similar to that employed in producing the (i) W/O emulsion above.

Subsequently, the above (i) W/O emulsion or (ii) oil phase is added to an external water phase, dispersed and emulsified (secondary emulsification) by a homomixer, etc. to produce an emulsion (hereinafter, the emulsion obtained from the W/O emulsion is sometimes referred to as a W/O/W emulsion, whereas the emulsion obtained from the (ii) oil phase is sometimes referred to as an O/W emulsion).

The amount of external water phase to be used is usually about 1 to about 10,000 fold by volume relative to the W/O emulsion or oil phase, preferably about 10 to about 5,000 fold by volume and particularly preferably about 50 to about 1,000 fold by volume.

To the external water phase, an emulsifier is usually added. As the emulsifier, any emulsifier can be used as long as it can usually form a stable W/O/W emulsion or O/W emulsion. Examples thereof include an anionic surfactant, a nonionic surfactant, a polyoxyethylene castor oil derivative, polyvinylpyrrolidone, polyvinyl alcohol, carboxymethylcellulose, lecithin, gelatin and hyaluronic acid. Among others, polyvinyl alcohol is preferable. The concentration of an emulsifier in an external water phase is usually about 0.001 to about 20% (w/w), preferably about 0.01 to about 10% (w/w) and particularly preferably about 0.05 to about 5% (w/w).

The W/O/W emulsion or O/W emulsion (hereinafter, these each may sometimes simply be referred to as an emulsion) thus obtained is subjected to an in-water-drying method to remove an organic solvent contained in the emulsion. In this manner, microcapsules can be produced.

Furthermore, other than the method using the aforementioned W/O/W emulsion or O/W emulsion, there is a production method in which an S (solid phase)/O (oil phase) emulsion, which is composed of a solid phase containing compound (I) or a salt thereof and a lactic acid polymer or a salt thereof, is subjected to an in-water-drying method.

First, a lactic acid polymer or a salt thereof is dissolved in an organic solvent. In the resulting organic solvent solution, compound (I) or a salt thereof is dispersed. At this time, the amounts of compound (I) or a salt thereof and lactic acid polymer or a salt thereof to be used may be selected such that the ratio of compound (I) or a salt thereof to the lactic acid polymer or a salt thereof becomes the same as in producing the above (i) W/O emulsion. Furthermore, to uniformly disperse compound (I) or a salt thereof in the organic solvent, for example, ultrasonic irradiation, a turbine-form stirrer or a homogenizer, is used.

Next, the S/O emulsion thus prepared is further added to an external water phase and dispersed and emulsified by use of, for example, ultrasonic irradiation, a turbine-form stirrer or a homogenizer to produce an emulsion (hereinafter sometimes referred to as an S (solid phase)/O (oil phase)/W (water phase) emulsion). Thereafter, the oil-phase solvent is vaporized to produce microcapsules. At this time, the volume of the water phase is generally selected from about 1 fold to about 10,000 fold of the oil phase by volume, further preferably about 10 fold to about 5,000 fold and particularly preferably about 50 fold to about 1,000 fold.

To the external water phase, an emulsifier as mentioned above may be added. The amount of external water phase to be added and the type and concentration of emulsifier to be added to the external water phase are the same as those employed in producing the above W/O/W emulsion.

The S/O/W emulsion thus obtained is subjected to an in-water-drying method to remove an organic solvent. In this manner, microcapsules can be produced.

The microcapsules obtained by using a W/O/W emulsion, an O/W emulsion, an S/O/W emulsion were separated by centrifugation, sieving or filtration, and then, if necessary, washed with distilled water to remove an emulsifier, etc. attached to the surface of microcapsules. Thereafter, the microcapsules are dispersed in distilled water, etc., lyophilized, and, if necessary, warmed to further remove water and an organic solvent in the microcapsule. Warming may be performed under reduced pressure. As the conditions for a warming step, heat dry is performed at a temperature, which is not less than a glass-transition temperature of the lactic acid polymer or a salt thereof used herein and at which microcapsule particles are not adhered to each other. Preferably, heat dry is performed in the temperature range from the glass-transition temperature of a lactic acid polymer or a salt thereof to a temperature higher by about 30°C thereof. The glass-transition temperature used herein refers to a medium point of the temperatures obtained by measuring using a differential scanning calorimeter at a temperature raising rate of 10 to 20°C/minute.

(2-b) Phase separation method

When microcapsules are produced by this method, a coacervation agent is gradually added under stirring to the W/O emulsion described in the above (2-a) "in-water-drying method". In this manner, microcapsules are precipitated and solidified. The amount of coacervation agent is selected from about 0.01 to about 1,000 fold of oil phase volume, preferably about 0.05 to about 500 fold and particularly preferably about 0.1 to about 200 fold.

The coacervation agent is not particularly limited as long as it is a polymer compound, a mineral-oil based compound or a vegetable-oil based compound, etc. that is miscible with an organic solvent and does not dissolve a biodegradable polymer of the present invention. Specific examples thereof that may be used include silicon oil, sesame oil, soybean oil, corn oil, cotton seed oil, coconut oil, linseed oil, mineral oil, n-hexane and n-heptane. These may be used as a mixture of two types or more.

After the microcapsules thus obtained are separated, they are repeatedly washed with heptane, etc. to remove the coacervation agent except a physiologically active substance and a composition formed of a biodegradable polymer of the present invention and dried under reduced pressure. Alternatively, washing is performed in the same manner as described in the above (2-a) "in-water-drying method", followed by lyophilizing and warm-drying.

(2-c) Spray drying method

When microcapsules are produced by this method, the W/O emulsion described in the above (2-a) "in-water-drying method" is sprayed by a nozzle in a dehydration chamber of a spray drier to volatilize the organic solvent within micro liquid drops within an extremely short time to produce microcapsules. Examples of the nozzle include a two-fluid nozzle type, a pressure nozzle type and a rotation disk type. Thereafter, if necessary, washing is performed in the same manner as described in the above (2-a) "in-water-drying method" and thereafter may be lyophilized and further dried by warming.

As the dosage form other than the aforementioned microcapsule, microparticles may be mentioned, which are obtained by drying the W/O emulsion described in the above (2-a) "in-water-drying method", for example, by a rotary evaporator, into a solid, while vaporizing an organic solvent and water by controlling the degree of vacuum, and thereafter pulverizing by a jet mill, etc.

Furthermore, the microparticles pulverized are washed in the same manner as described in the above (2-a) "in-water-drying method" and thereafter may be lyophilized and further dried by warming.

In dispersing the microcapsules produced in the above section (2-a), (2-b) or (2-c) in distilled water, etc., an aggregation preventing agent may be added in order to prevent aggregation of particles. Examples of the aggregation preventing agent include water soluble polysaccharides such as mannitol, lactose, glucose, a starch (for example, cornstarch) and hyaluronic acid or an alkali metal salt thereof; proteins such as glycine, fibrin and collagen; and inorganic salts such as sodium chloride and sodium hydrogenphosphate. Among others, mannitol is preferable. The amount of aggregation preventing agent to be used is preferably about 2 to about 100 parts by weight relative to microcapsule (100 parts by weight) and further preferably about 10 to about 25 parts by weight.

Furthermore, microcapsules may be warmed and then cooled in the same manner as described in the case of the above (1-a) to obtain moldings of disk form, film form and rod form, etc.

The content of compound (I) or a salt thereof in a microcapsule is not particularly limited; however, the content of a 3-month sustained-release formulation is, for example, 4% or more to 10% or less and preferably 6% or more to 10% or less. Furthermore, the content of a 6-month sustained-release formulation is, for example, 4% or more to 18% or less, preferably 6% or more to 16% or less and more preferably 7% or more to 10% or less.

Of the various production methods mentioned above, a production method, which includes subjecting an O/W emulsion obtained by emulsifying an oil phase containing compound (I) or a salt thereof and a lactic acid polymer or a salt thereof, to an in-water drying method is preferred, in view of the sustained release period of the sustained-release formulation of the present invention.

In various production methods as mentioned above, in dissolving a lactic acid polymer or a salt thereof in an organic solvent, zinc oxide may be added to the organic solvent.

The amount of zinc oxide to be used is, for example, about 0.01 to about 100 parts by weight relative to a lactic acid polymer (100 parts by weight), preferably about 0.1 to about 20 parts by weight.

Furthermore, the particle size of zinc oxide is usually about 0.001 to about 10 $\mu$m and preferably about 0.005 to about 1 $\mu$m.

Likewise, the sustained-release formulation obtained by using zinc oxide has excellent properties, such as "high uptake rate of a drug", "ability to persistently release a drug over a long period of time", etc.

In producing the sustained-release formulation of the present invention, compound (I) or a salt thereof may be dissolved in an aqueous solution of a volatile salt, for example, ammonium acetate, lyophilized and then put in use.

The lyophilized product of compound (I) or a salt thereof obtained by treating with ammonium acetate in this way has a small particle size and excellent operability, and thus advantageous in producing a sustained-release formulation.

The sustained-release formulation of the present invention thus obtained, if desired, may appropriately contain pharmaceutically acceptable additives (for example, a stabilizer, a preservative, a soothing agent). Examples of the dosage form of the sustained-release formulation of the present invention include parenteral agents (for example, an injection, an implantation, a suppository) and oral administration agents (for example, a solid formulation such as a capsule agent, a tablet, a granule and a powder; liquid formulation such as a syrup, an emulsion and a suspension). Examples of the stabilizer include human serum albumin and polyethylene glycol. Examples of the preservative include benzyl alcohol and phenol. Examples of the soothing agent include benzalkonium chloride and procaine hydrochloride. In the sustained-release formulation of the present invention, the content of compound (I) or a salt thereof can be usually and appropriately selected within the range of about 0.01 to about 33% (w/w) relative to the total sustained-release formulation.

The sustained-release formulation of the present invention is excellent in that a blood drug concentration of compound (I) or a salt thereof is stable in a sustained-release period.

The sustained-release formulation of the present invention is preferably a parenteral agent and further preferably an injection. For example, when the sustained-release formulation is in the form of microcapsules, the microcapsules are used in combination with a dispersant (e.g., a surfactant such as Tween 80 and HCO-60; and a polysaccharide such as carboxymethylcellulose, sodium alginate and hyaluronic acid), a preservative (e.g., methylparaben and propylparaben) and an isotonic agent (e.g., sodium chloride, mannitol, sorbitol and glucose) etc. to prepare an aqueous suspension. In this manner, a sustained-release injection can be obtained. Furthermore, a sustained-release injection can be obtained also by dispersing microcapsules in a vegetable oil such as sesame oil and corn oil or in the vegetable oil to which a phospholipid such as lecithin is added, or in a medium chain triglyceride (e.g., Miglyol 812) to obtain an oily suspension.

When the sustained-release formulation is, for example, in the form of microcapsules, the particle size of microcapsules that are used as a suspension injection may be satisfactory if it satisfies the polydispersity and the range passing through a syringe needle. As an average particle size thereof, for example, the range of about 0.1 to about 300 $\mu$m may be mentioned. The average particle size thereof preferably falls within the range of about 1 to about 150 $\mu$m and particularly preferably about 2 to about 100 $\mu$m.

The aforementioned microcapsules are aseptically treated by a method of performing the whole production steps in aseptic conditions, a method of sterilizing with gamma ray and a method of adding an aseptic agent. The method is not particularly limited.

Since the sustained-release formulation of the present invention is low in its toxicity, it can be safely administered orally or parenterally to mammals (for example, human, monkey, hamadryas, chimpanzee, pig, cow, sheep, horse, dog, cat, mouse, rat).

The sustained-release formulation of the present invention can be used for treating or preventing all diseases in which a physiological activity of metastin is involved. In particular, the sustained-release formulation of the present invention can be effectively used in treating or preventing cancer (for example, lung cancer, stomach cancer, liver cancer, pancreatic cancer, large bowel cancer, rectal cancer, colon cancer, prostatic cancer, ovary cancer, uterine cervix cancer, breast cancer, kidney cancer, bladder cancer, brain tumor), a pancreatic disease (for example, acute or chronic pancreatitis), chorioma, hydatidiform mole, invasive mole, miscarriage, fetus hypogenesis, anomaly of saccharometabolism, lipidosis and abnormal childbirth.

The sustained-release formulation of the present invention is particularly useful as a therapeutic agent or prophylactic agent for cancer (preferably prostatic cancer).

The dose of the sustained-release formulation of the present invention can be appropriately selected depending upon the type and content of an active ingredient, i.e., compound (I) or a salt thereof, dosage form, duration of release, a subject for administration, administration route, administration purpose, target disease and symptom, etc.; however, the dose may be satisfactory as long as the active ingredient can be maintained in a living body in a pharmaceutically effective concentration in a desired duration. For example, in the therapy for an adult cancer patient, when the sustained-release formulation of the present invention is administered, for example, via injection for an about 1-month sustained-release, compound (I) or a salt thereof is used in an amount of, for example, within the range of about 0.01 to about 4 mg/kg body weight, and preferably about 0.03 to 0.6 mg/kg body weight per administration. Furthermore, when the sustained-release formulation of the present invention is administered via injection for an about 3-month sustained-release, compound (I) or a salt thereof is used in an amount of, for example, within the range of about 0.03 to about 12 mg/kg body weight, and preferably about 0.09 to about 1.8 mg/kg body weight per administration. Moreover, when the sustained-release formulation of the present invention is administered via injection for an about 6-month sustained-release, compound (I) or a salt thereof is used in an amount of, for example, within the range of about 0.06 to about 24 mg/kg body weight, and preferably about 0.18 to about 3.6 mg/kg body weight per administration. The administration frequency is, for example, once per month, once per 3 months, once per 6 months and can be appropriately selected depending upon the content of compound (I) or a salt thereof, dosage form, duration of release, a target disease and a subject for administration, etc. As the sustained-release formulation of the present invention, preferably a 1 to 8-month sustained-release formulation (that is, a formulation slowly releasing compound (I) or a salt thereof during the period of 1 to 8 months), more preferably a 1 to 6-month sustained-release formulation, further preferably a 3 to 6-month sustained release formulation and further more preferably a 6-month sustained release formulation is used.

Furthermore, the sustained-release formulation of the present invention can be used in combination with other medicines (hereinafter, simply referred to as a combined medicine) for various diseases for which compound (I) or a salt thereof pharmaceutically effectively works, in particular, medicinal agents such as a chemotherapeutic agent, a hormonal therapeutic agent and an immunotherapeutic agent for cancer treatment. In such cases, the administration periods of the sustained-release formulation of the present invention and the combined medicine are not limited. They can be administered to a subject for administration simultaneously or at a time interval. The dosage amount of combined medicine can be appropriately selected based on clinical dosage amount. Furthermore, a blending ratio of the sustained-release formulation of the present invention and a combined medicine can be appropriately selected depending upon a subject for administration, administration route, target disease, symptom and combination, etc.

Examples of the chemotherapeutic agent include alkylating agents (for example, cyclophosphamide, ifosfamide, nimustine, ranimustine, carboquone), antimetabolites (for example, methotrexate, 5-fluorouracil, tegafur, carmofur, UFT, doxifluridine, cytarabine, enocitabine, mercaptopurine, mercaptopurine riboside, thioguanine), anticancer antibiotic substances (for example, mitomycin, adriamycin, daunorubicin, epirubicin, pirarubicin, idarubicin, bleomycin, peplomycin, actinomycin) and plant-derived anticancer agents (for example, vincristine, vinblastine, vindesine, etoposide, camptothecine, irinotecan), cisplatin, carboplatin, nedaplatin, paclitaxel, docetaxel and estramustine.

Examples of the hormonal therapeutic agent include, adrenocortical hormones (for example, prednisolone, prednisone, dexamethasone, cortisone acetate), estrogens (for example, estradiol, ethinylestradiol, fosfestrol, chlorotrianisene), antiestrogen (for example, epitiostanol, mepitiostane, tamoxifen, clomiphene), progesterons (for example, hydroxyprogesterone caproate, dydrogesterone, medroxyprogesterone, norethisterone, norethindrone) and LHRH derivatives (for example, leuprorelin acetate).

Examples of the immunotherapeutic agent include microbial or bacterial components (for example, a muramyldipeptide derivative, picibanil), polysaccharides having an immunological-enhancing activity (for example, lentinan, sizofiran, krestin), cytokines obtained by a genetic engineering approach (for example, interferon, interleukin 2(IL-2), interleukin 12 (IL-12), tumor necrosis factor (TNF)) and colony stimulating factors (for example, granulocyte colony stimulating factor, erythropoietin).

Furthermore, medicines that are confirmed to have an effect of ameliorating cachexia in animal models or clinical practice; more specifically, cyclooxygenase inhibitors (for example, indomethacin) [Cancer Research, Vol. 49, pages 5935 to 5939, 1989], progesterone derivatives (for example, megesterol acetate)[Journal of Clinical Oncology, Vol. 12, pages 213 to 225, 1994], glucocorticosteroids (for example, dexamethasone), metoclopramide based medicines, tetrahydrocannabinol based medicines (literatures are the same as mentioned above), fat metabolism improving agents (for example, eicosapentaenoic acid)[British Journal of Cancer, Vol. 68, pages 314 to 318, 1993], growth hormone, IGF-1, or antibodies against a factor of inducing cachexia, i.e., TNF-$\alpha$, LIF, IL-6, oncostatin M can be used in combination with the sustained-release formulation of the present invention.

Other than these, general medicines for use in treating or preventing diseases of the placenta and pancreas can be used as combined medicines. Examples of such medicines include an anti-inflammatory agent, an antipyretic/analgesic agent, an antibacterial agent, an antiviral agent and a hormonal agent that are clinically used in general.

In the specification, when bases and amino acids, etc. are expressed by abbreviations, they are expressed based on IUPAC IUB Commission on Biochemical Nomenclature or conventional abbreviations routinely used in the art. Examples thereof are as follows. When an optical isomer of an amino acid is conceivably present, unless otherwise specified, an L-form amino acid is shown.

| | |
|---|---|
| Ac | : acetyl |
| AzaGly | : azaglycine |
| Hyp | : trans-4-hydroxyproline |
| Leu | : leucine |
| Thr | : threonine |
| Arg(Me) | : Nω-methyl arginine |
| Phe | : phenylalanine |
| Tyr | : tyrosine |
| Trp | : tryptophan |
| Asn | : asparagine |

## 5. EXAMPLES

The present invention will be more specifically explained below by way of Examples and Test Examples.

Except for the active component, substances described in, e.g., the Japanese Pharmacopeia, 15th revision, Japanese Standards for Pharmaceutical Ingredients, or adaptive substances listed in the standard for pharmaceutical additive 2003 were used as components (i.e., additives) for the prescriptions described as Examples below.

Example 1

A lactic acid polymer (weight average molecular weight Mw: 6,500, number average molecular weight Mn: 2,800, Mw/Mn ratio: 2.3; manufactured by Wako Pure Chemical Industries Ltd.) (7.0417 g) was dissolved in dichloromethane (13.186 g). This solution (15.56 g) was weighed and blended with a solution prepared by dissolving an acetate of compound (I) (0.7406 g) in methanol (2.819 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7,000 rpm). The O/W emulsion was stirred for about 3 hours (in-water-drying step) and sieved by use of a 75 μm standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.855 g) was added. The mixture was lyophilized by a lyophilizer (DF-01H, ULVAC) to obtain microcapsule powder. The content of compound (I) in the resulting microcapsule powder was 8.1 %.

Example 2

A lactic acid polymer (weight average molecular weight Mw: 8,000, number average molecular weight Mn: 3,400, Mw/Mn ratio: 2.4; manufactured by Wako Pure Chemical Industries Ltd.) (7.0530 g) was dissolved in dichloromethane (13.269 g). This solution (15.82 g) was weighed and blended with a solution prepared by dissolving an acetate of compound (I) (0.7398 g) in methanol (2.832 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7,000 rpm). The O/W emulsion was stirred for about 3 hours (in-water-drying method step) and sieved by use of a 75 μm standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.866 g) was added. The mixture was lyophilized by a lyophilizer (DF-01H, ULVAC) to obtain microcapsule powder. The content of compound (I) in the resulting microcapsule powder was 8.4%.

Example 3

A lactic acid polymer (weight average molecular weight Mw: 10,000, number average molecular weight Mn: 4,000, Mw/Mn ratio: 2.5; manufactured by Wako Pure Chemical Industries Ltd.) (7.0405 g) was dissolved in dichloromethane (13.184 g). This solution (15.54 g) was weighed and blended with a solution prepared by dissolving an acetate of compound (I) (0.7397 g) in methanol (2.82 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7,000 rpm). The O/W emulsion was stirred for about 3 hours (in-water-drying method step) and sieved by use of a 75 $\mu$m standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.840 g) was added. The mixture was lyophilized by a lyophilizer (DF-01H, ULVAC) to obtain microcapsule powder. The content of compound (I) in the resulting microcapsule powder was 8.6%.

Example 4

A lactic acid polymer (weight average molecular weight Mw: 11,800, number average molecular weight Mn: 4,900, Mw/Mn ratio: 2.4; manufactured by Wako Pure Chemical Industries Ltd.) (13.3028 g) was dissolved in dichloromethane (24.15 g). This solution (15.60 g) was weighed and blended with a solution prepared by dissolving an acetate of compound (I) (0.7417 g) in methanol (2.83 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7,000 rpm). The O/W emulsion was stirred for about 3 hours (in-water-drying method step) and sieved by use of a 75 $\mu$m standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.852 g) was added. The mixture was lyophilized by a lyophilizer (DF-01H, ULVAC) to obtain microcapsule powder. The content of compound (I) in the resulting microcapsule powder was 8.6%.

Example 5

A lactic acid polymer (weight average molecular weight Mw: 8,000, number average molecular weight Mn: 3,400, Mw/Mn ratio: 2.4; manufactured by Wako Pure Chemical Industries Ltd.) (13.3374 g) was dissolved in dichloromethane (24.47 g). This solution (15.54 g) was weighed and blended with an aqueous solution prepared by dissolving an acetate of compound (I) (0.7450 g) in distilled water (0.60 g) and emulsified by a small homogenizer (KINEMATICA) to form a W/O emulsion (rotation rate: about 10,000 rpm, 30 sec). Subsequently, the W/O emulsion was adjusted to 32°C and poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to obtain a W/O/W emulsion (evolution of turbine: about 7,000 rpm). The W/O/W emulsion was stirred for about 3 hours (in-water-drying method step) and sieved by use of a 75 $\mu$m standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.872 g) was added. The mixture was lyophilized by a lyophilizer (DF-01H, ULVAC) to obtain microcapsule powder. The content of compound (I) in the resulting microcapsule powder was 7.2%.

Example 6

A lactic acid polymer (weight average molecular weight Mw: 10,000, number average molecular weight Mn: 4,000, Mw/Mn ratio: 2.5; manufactured by Wako Pure Chemical Industries Ltd.) (13.3334 g) was dissolved in dichloromethane (24.30 g). This solution (15.90 g) was weighed and blended with an aqueous solution prepared by dissolving an acetate of compound (I) (0.7554 g) in distilled water (0.60 g) and emulsified by a small homogenizer (KINEMATICA) to form a W/O emulsion (rotation rate: about 10,000 rpm, 30 sec). Subsequently, the W/O emulsion was adjusted to 32°C and poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to obtain a W/O/W emulsion (evolution of turbine: about 7,000 rpm). The W/O/W emulsion was stirred for about 3 hours (in-water-drying method step) and sieved by use of a 75 μm standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.864 g) was added. The mixture was lyophilized by a lyophilizer (DF-01H, ULVAC) to obtain microcapsule powder. The content of compound (I) in the resulting microcapsule powder was 7.2%.

Example 7

A lactic acid polymer (weight average molecular weight Mw: 11,800, number average molecular weight Mn: 4,900, Mw/Mn ratio: 2.4; manufactured by Wako Pure Chemical Industries Ltd.) (13.2307 g) was dissolved in dichloromethane (24.74 g). This solution (15.57 g) was weighed and blended with an aqueous solution prepared by dissolving an acetate of compound (I) (0.7589 g) in distilled water (0.60 g) and emulsified by a small homogenizer (KINEMATICA) to form a W/O emulsion (rotation rate: about 10,000 rpm, 30 sec). Subsequently, the W/O emulsion was adjusted to 32°C and poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to obtain a W/O/W emulsion (evolution of turbine: about 7,000 rpm). The W/O/W emulsion was stirred for about 3 hours (in-water-drying method step) and sieved by use of a 75 μm standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.845 g) was added. The mixture was lyophilized by a lyophilizer (DF-01H, ULVAC) to obtain microcapsule powder. The content of compound (I) in the resulting microcapsule powder was 6.8%.

Example 8

A lactic acid polymer (weight average molecular weight Mw: 6,500, number average molecular weight Mn: 2,800, Mw/Mn ratio: 2.3; manufactured by Wako Pure Chemical Industries Ltd.) (6.2666 g) was dissolved in dichloromethane (10.975 g). This solution (13.20 g) was weighed and blended with a solution prepared by dissolving an acetate of compound (I) (1.4825 g) in methanol (5.61 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7,000 rpm). The O/W emulsion was stirred for about 3 hours (in-water-drying method step) and sieved by use of a 75 μm standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.851 g) was added. The mixture was lyophilized by a lyophilizer (DF-01H, ULVAC) to obtain microcapsule powder. The content of compound (I) in the resulting microcapsule powder was 16.4%.

Example 9

A lactic acid polymer (weight average molecular weight Mw: 8,000, number average molecular weight Mn: 3,400, Mw/Mn ratio: 2.4; manufactured by Wako Pure Chemical Industries Ltd.) (6.2621 g) was dissolved in dichloromethane (10.948 g). This solution (13.22 g) was weighed and blended with a solution prepared by dissolving an acetate of compound (I) (1.4795 g) in methanol (5.60 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7,000 rpm). The O/W emulsion was stirred for about 3 hours (in-water-drying method step) and sieved by use of a 75 μm standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.854 g) was added. The mixture was lyophilized by a lyophilizer (DF-01H, ULVAC) to obtain microcapsule powder. The content of compound (I) in the resulting microcapsule powder was 16.4%.

Example 10

A lactic acid polymer (weight average molecular weight Mw: 10,000, number average molecular weight Mn: 4,000, Mw/Mn ratio: 2.5; manufactured by Wako Pure Chemical Industries Ltd.) (6.2617 g) was dissolved in dichloromethane (10.971 g). This solution (13.18 g) was weighed and blended with a solution prepared by dissolving an acetate of compound (I) (1.4775 g) in methanol (5.63 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7,000 rpm). The O/W emulsion was stirred for about 3 hours (in-water-drying method step) and sieved by use of a 75 $\mu$m standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.848 g) was added. The mixture was lyophilized by a lyophilizer (DF-01H, ULVAC) to obtain microcapsule powder. The content of compound (I) in the resulting microcapsule powder was 15.5%.

Example 11

A lactic acid polymer (weight average molecular weight Mw: 11,800, number average molecular weight Mn: 4,900, Mw/Mn ratio: 2.4; manufactured by Wako Pure Chemical Industries Ltd.) (13.3028 g) was dissolved in dichloromethane (24.15 g). This solution (13.46 g) was weighed and blended with a solution prepared by dissolving an acetate of compound (I) (1.4821 g) in methanol (5.75 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7,000 rpm). The O/W emulsion was stirred for about 3 hours (in-water-drying method step) and sieved by use of a 75 $\mu$m standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.857 g) was added. The mixture was lyophilized by a lyophilizer (DF-01H, ULVAC) to obtain microcapsule powder. The content of compound (I) in the resulting microcapsule powder was 13.3%.

Example 12

A lactic acid polymer (weight average molecular weight Mw: 8,000, number average molecular weight Mn: 3,400, Mw/Mn ratio: 2.4; manufactured by Wako Pure Chemical Industries Ltd.) (13.3374 g) was dissolved in dichloromethane (24.47 g). This solution (13.42 g) was weighed and blended with an aqueous solution prepared by dissolving an acetate of compound (I) (1.4892 g) in distilled water (1.20 g) and emulsified by a small homogenizer (KINEMATICA) to form a W/O emulsion (rotation rate: about 10,000 rpm, 30 sec). Subsequently, the W/O emulsion was adjusted to 32°C and poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to obtain a W/O/W emulsion (evolution of turbine: about 7,000 rpm). The W/O/W emulsion was stirred for about 3 hours (in-water-drying method step) and sieved by use of a 75 $\mu$m standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.878 g) was added. The mixture was lyophilized by a lyophilizer (DF-01H, ULVAC) to obtain microcapsule powder. The content of compound (I) in the resulting microcapsule powder was 13.1%.

Example 13

A lactic acid polymer (weight average molecular weight Mw: 10,000, number average molecular weight Mn: 4,000, Mw/Mn ratio: 2.5; manufactured by Wako Pure Chemical Industries Ltd.) (13.3334 g) was dissolved in dichloromethane (24.30 g). This solution (13.38 g) was weighed and blended with an aqueous solution prepared by dissolving an acetate of compound (I) (1.4866 g) in distilled water (1.21 g) and emulsified by a small homogenizer (KINEMATICA) to form a W/O emulsion (rotation rate: about 10,000 rpm, 30 sec). Subsequently, the W/O emulsion was adjusted to 32°C and poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to obtain a W/O/W emulsion (evolution of turbine: about 7,000 rpm). The W/O/W emulsion was stirred for about 3 hours (in-water-drying method step) and sieved by use of a 75 μm standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.885 g) was added. The mixture was lyophilized by a lyophilizer (DF-01H, ULVAC) to obtain microcapsule powder. The content of compound (I) in the resulting microcapsule powder was 12.6%.

Example 14

A lactic acid polymer (weight average molecular weight Mw: 11,800, number average molecular weight Mn: 4,900, Mw/Mn ratio: 2.4; manufactured by Wako Pure Chemical Industries Ltd.) (13.2307 g) was dissolved in dichloromethane (24.74). This solution (13.43 g) was weighed and blended with an aqueous solution prepared by dissolving an acetate of compound (I) (1.4900 g) in distilled water (1.21 g) and emulsified by a small homogenizer (KINEMATICA) to form a W/O emulsion (rotation rate: about 10,000 rpm, 30 sec). Subsequently, the W/O emulsion was adjusted to 32°C and poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to obtain a W/O/W emulsion (evolution of turbine: about 7,000 rpm). The W/O/W emulsion was stirred for about 3 hours (in-water-drying method step) and sieved by use of a 75 μm standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.885 g) was added. The mixture was lyophilized by a lyophilizer (DF-01H, ULVAC) to obtain microcapsule powder. The content of compound (I) in the resulting microcapsule powder was 13.0%.

Example 15

A lactic acid polymer (weight average molecular weight Mw: 14,300, number average molecular weight Mn: 5,400, Mw/Mn ratio: 2.7; manufactured by Wako Pure Chemical Industries Ltd.) (6.24 g) was dissolved in dichloromethane (10.92 g). This solution (13.28 g) was weighed and blended with a solution prepared by dissolving an acetate of compound (I) (1.3619 g) in methanol (5.73 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7,000 rpm). The O/W emulsion was stirred for about 3 hours (in-water-drying method step) and sieved by use of a 75 μm standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.740 g) was added. The mixture was lyophilized by a lyophilizer (DF-01H, ULVAC) to obtain microcapsule powder. The content of compound (I) in the resulting microcapsule powder was 15.1%.

Example 16

A lactic acid polymer (weight average molecular weight Mw: 16,000, number average molecular weight Mn: 6,000, Mw/Mn ratio: 2.7; manufactured by Wako Pure Chemical Industries Ltd.) (6.26 g) was dissolved in dichloromethane (10.98 g). This solution (13.27 g) was weighed and blended with a solution prepared by dissolving an acetate of compound (I) (1.3627 g) in methanol (5.61 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7,000 rpm). The O/W emulsion was stirred for about 3 hours (in-water-drying method step) and sieved by use of a 75 μm standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.740 g) was added. The mixture was lyophilized by a lyophilizer (DF-01H, ULVAC) to obtain microcapsule powder. The content of compound (I) in the resulting microcapsule powder was 15.7%.

Example 17

A lactic acid polymer (weight average molecular weight Mw: 14,300, number average molecular weight Mn: 5,400, Mw/Mn ratio: 2.7; manufactured by Wako Pure Chemical Industries Ltd.) (7.02 g) was dissolved in dichloromethane (13.18 g). This solution (15.64 g) was weighed and blended with a solution prepared by dissolving an acetate of compound (I) (0.6799 g) in methanol (5.57 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7,000 rpm). The O/W emulsion was stirred for about 3 hours (in-water-drying method step) and sieved by use of a 75 μm standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.740 g) was added. The mixture was lyophilized by a lyophilizer (DF-01H, ULVAC) to obtain microcapsule powder. The content of compound (I) in the resulting microcapsule powder was 8.4%.

Example 18

A lactic acid polymer (weight average molecular weight Mw: 16,000, number average molecular weight Mn: 6,000, Mw/Mn ratio: 2.7; manufactured by Wako Pure Chemical Industries Ltd.) (7.01 g) was dissolved in dichloromethane (13.11 g). This solution (15.50 g) was weighed and blended with a solution prepared by dissolving an acetate of compound (I) (0.6870 g) in methanol (5.61 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7,000 rpm). The O/W emulsion was stirred for about 3 hours (in-water-drying method step) and sieved by use of a 75 μm standard sieve, and then, microspheres were collected by centrifugation (rotation rate: about 2,500 rpm, 5 min) using a centrifuge (HIMAC CR 5DL, manufactured by Hitachi, Ltd.). This was dispersed again in distilled water and further centrifuged to wash away free substances, etc. The collected microspheres were redispersed in a small amount of distilled water and mannitol (0.768 g) was added. The mixture was lyophilized by a lyophilizer (DF-01H, ULVAC) to obtain microcapsule powder. The content of compound (I) in the resulting microcapsule powder was 9.2%.

Comparative Example 1

In the case where a solution mixture of a solution prepared by dissolving a lactic acid polymer in dichloromethane and an aqueous solution prepared by dissolving an acetate of compound (II) (Ac-D-Tyr-D-Trp-Asn-Thr-Phe-AzaGly-Leu-Arg(Me)-Trp-NH$_2$) (SEQ ID NO: 2) in distilled water, is allowed to suspend by a mini-mixer, the solution mixture is gelatinized. Therefore, a W/O emulsion cannot be obtained.

Test Example 1

The microcapsule powder (9.6 mg as a free compound (I)) of Example 15 was dispersed in a dispersion medium (0.9 mL) (a solution in which carboxymethylcellulose (5.50 mg), polysorbate 80 (0.90 mg) and mannitol (45.0 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) over a period of about 22 weeks was demonstrated.

Test Example 2

The microcapsule powder (9.6 mg as a free compound (I)) of Example 16 was dispersed in a dispersion medium (0.9 mL) (a solution in which carboxymethylcellulose (5.50 mg), polysorbate 80 (0.90 mg) and mannitol (45.0 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) over a period of about 24 weeks was demonstrated.

Test Example 3

The microcapsule powder (9.6 mg as a free compound (I)) of Example 17 was dispersed in a dispersion medium (0.9 mL) (a solution in which carboxymethylcellulose (5.50 mg), polysorbate 80 (0.90 mg) and mannitol (45.0 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) over a period of about 22 weeks was demonstrated.

Test Example 4

The microcapsule powder (4.8 mg as a free compound (I)) of Example 5 was dispersed in a dispersion medium (0.9 mL) (a solution in which carboxymethylcellulose (5.50 mg), polysorbate 80 (0.90 mg) and mannitol (45.0 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) over a period of about 15 weeks was demonstrated.

Test Example 5

The microcapsule powder (4.8 mg as a free compound (I)) of Example 6 was dispersed in a dispersion medium (0.9 mL) (a solution in which carboxymethylcellulose (5.50 mg), polysorbate 80 (0.90 mg) and mannitol (45.0 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) over a period of about 15 weeks was demonstrated.

Test Example 6

The microcapsule powder (4.8 mg as a free compound (I)) of Example 7 was dispersed in a dispersion medium (0.9 mL) (a solution in which carboxymethylcellulose (5.50 mg), polysorbate 80 (0.90 mg) and mannitol (45.0 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) over a period of about 18 weeks was demonstrated.

**Claims**

1. A sustained-release formulation comprising a compound represented by Formula:

$$Ac\text{-}D\text{-}Tyr\text{-}Hyp\text{-}Asn\text{-}Thr\text{-}Phe\text{-}AzaGly\text{-}Leu\text{-}Arg(Me)\text{-}Trp\text{-}NH_2$$

or a salt thereof and a lactic acid polymer having a weight average molecular weight of about 5,000 to about 40,000 or a salt thereof, wherein the lactic acid polymer is a polymer consisting of lactic acid alone.

2. The sustained-release formulation according to claim 1, wherein the weight average molecular weight of the lactic acid polymer or a salt thereof is about 13,000 to about 17,000.

3. The sustained-release formulation according to claim 1, which is a 3 to 6-month sustained-release formulation.

4. The sustained-release formulation according to claim 1, which is a therapeutic or prophylactic agent for cancer.

5. The sustained-release formulation according to claim 1, which is a parenteral agent.

6. A sustained-release formulation according to clam 1 for use in treating or preventing cancer.

7. A method for producing the sustained-release formulation according to claim 1, comprising subjecting

   (1) a W/O/W emulsion obtained by emulsifying a W/O emulsion composed of an internal water phase containing a compound represented by Formula:

   $$Ac\text{-}D\text{-}Tyr\text{-}Hyp\text{-}Asn\text{-}Thr\text{-}Phe\text{-}AzaGly\text{-}Leu\text{-}Arg(Me)\text{-}Trp\text{-}NH_2$$

   or a salt thereof, and an oil phase containing the lactic acid polymer or a salt thereof,
   or
   (2) an O/W emulsion obtained by emulsifying an oil phase composed of a compound represented by Formula:

   $$Ac\text{-}D\text{-}Tyr\text{-}Hyp\text{-}Asn\text{-}Thr\text{-}Phe\text{-}AzaGly\text{-}Leu\text{-}Arg(Me)\text{-}Trp\text{-}NH_2$$

   or a salt thereof and the lactic acid polymer or a salt thereof; to an in-water drying method.

8. The method according to claim 7, comprising subjecting an O/W emulsion obtained by emulsifying an oil phase containing a compound represented by Formula:

   $$Ac\text{-}D\text{-}Tyr\text{-}Hyp\text{-}Asn\text{-}Thr\text{-}Phe\text{-}AzaGly\text{-}Leu\text{-}Arg(Me)\text{-}Trp\text{-}NH_2$$

   or a salt thereof and the lactic acid polymer or a salt thereof, to an in-water drying method.

**Patentansprüche**

1. Zubereitung mit nachhaltiger Wirkstofffreisetzung, umfassend eine Verbindung, die durch die Formel

   $$Ac\text{-}D\text{-}Tyr\text{-}Hyp\text{-}Asn\text{-}Thr\text{-}Phe\text{-}AzaGly\text{-}Leu\text{-}Arg(Me)\text{-}Trp\text{-}NH_2$$

dargestellt wird, oder ein Salz davon und ein Milchsäurepolymer mit einem Gewichtsmittel des Molekulargewichts von etwa 5000 bis etwa 40 000 oder ein Salz davon, wobei das Milchsäurepolymer ein Polymer ist, das nur aus Milchsäure besteht.

**2.** Zubereitung mit nachhaltiger Wirkstofffreisetzung gemäß Anspruch 1, wobei das Gewichtsmittel des Molekulargewichts des Milchsäurepolymers oder seines Salzes etwa 13 000 bis etwa 17 000 beträgt.

**3.** Zubereitung mit nachhaltiger Wirkstofffreisetzung gemäß Anspruch 1, bei der es sich um eine Zubereitung mit über 3 bis 6 Monate nachhaltiger Wirkstofffreisetzung handelt.

**4.** Zubereitung mit nachhaltiger Wirkstofffreisetzung gemäß Anspruch 1, bei der es sich um ein therapeutisches oder prophylaktisches Mittel gegen Krebs handelt.

**5.** Zubereitung mit nachhaltiger Wirkstofffreisetzung gemäß Anspruch 1, bei der es sich um ein parenterales Mittel handelt.

**6.** Zubereitung mit nachhaltiger Wirkstofffreisetzung gemäß Anspruch 1 zur Verwendung bei der Behandlung oder Prävention von Krebs.

**7.** Verfahren zur Herstellung der Zubereitung mit nachhaltiger Wirkstofffreisetzung gemäß Anspruch 1, umfassend das Durchführen eines In-Wasser-Trocknungsverfahrens mit:

(1) einer w/o/w-Emulsion, die dadurch erhalten wird, dass man eine w/o-Emulsion, die aus einer internen Wasserphase, welche eine durch die Formel

$$Ac\text{-}D\text{-}Tyr\text{-}Hyp\text{-}Asn\text{-}Thr\text{-}Phe\text{-}AzaGly\text{-}Leu\text{-}Arg(Me)\text{-}Trp\text{-}NH_2$$

dargestellte Verbindung oder ein Salz davon enthält, und einer Ölphase, welche das Milchsäurepolymer oder ein Salz davon enthält, besteht, emulgiert; oder
(2) einer o/w-Emulsion, die dadurch erhalten wird, dass man eine Ölphase, die aus einer durch die Formel

$$Ac\text{-}D\text{-}Tyr\text{-}Hyp\text{-}Asn\text{-}Thr\text{-}Phe\text{-}AzaGly\text{-}Leu\text{-}Arg(Me)\text{-}Trp\text{-}NH_2$$

dargestellten Verbindung oder einem Salz davon und dem Milchsäurepolymer oder einem Salz davon besteht, emulgiert.

**8.** Verfahren gemäß Anspruch 7, umfassend das Durchführen eines In-Wasser-Trocknungsverfahrens mit einer o/w-Emulsion, die dadurch erhalten wird, dass man eine Ölphase, die aus einer durch die Formel

$$Ac\text{-}D\text{-}Tyr\text{-}Hyp\text{-}Asn\text{-}Thr\text{-}Phe\text{-}AzaGly\text{-}Leu\text{-}Arg(Me)\text{-}Trp\text{-}NH_2$$

dargestellten Verbindung oder einem Salz davon und dem Milchsäurepolymer oder einem Salz davon besteht, emulgiert.

**Revendications**

**1.** Formulation à libération prolongée, comprenant un composé représenté par la formule

$$Ac\text{-}D\text{-}Tyr\text{-}Hyp\text{-}Asn\text{-}Thr\text{-}Phe\text{-}AzaGly\text{-}Leu\text{-}Arg(Me)\text{-}Trp\text{-}NH_2$$ ou un sel de ce composé, et un polymère d'acide lactique présentant une masse molaire moyenne en poids d'environ 5 000 à environ 40 000 ou un sel d'un tel polymère, lequel polymère d'acide lactique est un polymère constitué d'acide lactique seul.

**2.** Formulation à libération prolongée, conforme à la revendication 1, dans laquelle la masse molaire moyenne en poids du polymère d'acide lactique ou de son sel vaut d'environ 13 000 à environ 17 000.

**3.** Formulation à libération prolongée, conforme à la revendication 1, qui est une formulation à libération prolongée sur 3 à 6 mois.

**4.** Formulation à libération prolongée, conforme à la revendication 1, qui est un agent thérapeutique ou prophylactique contre un cancer.

**5.** Formulation à libération prolongée, conforme à la revendication 1, qui est un agent parentéral.

**6.** Formulation à libération prolongée, conforme à la revendication 1, pour utilisation dans le traitement ou la prévention d'un cancer.

**7.** Procédé de préparation d'une formulation à libération prolongée conforme à la revendication 1, comprenant le fait de soumettre à une opération de séchage dans l'eau :

1) une émulsion eau/huile/eau obtenue par émulsification d'une émulsion eau/huile, composée d'une phase aqueuse interne contenant un composé représenté par la formule

Ac-D-Tyr-Hyp-Asn-Thr-Phe-AzaGly-Leu-Arg(Me)-Trp-NH$_2$ ou un sel de ce composé, et d'une phase huileuse contenant un polymère d'acide lactique ou un sel d'un tel polymère ;

2) ou une émulsion huile/eau obtenue par émulsification d'une phase huileuse composée d'un composé représenté par la formule

Ac-D-Tyr-Hyp-Asn-Thr-Phe-AzaGly-Leu-Arg(Me)-Trp-NH$_2$

ou d'un sel de ce composé, et d'un polymère d'acide lactique ou d'un sel d'un tel polymère.

**8.** Procédé conforme à la revendication 7, comportant le fait de soumettre à une opération de séchage dans l'eau une émulsion huile/eau obtenue par émulsification d'une phase huileuse contenant un composé représenté par la formule

Ac-D-Tyr-Hyp-Asn-Thr-Phe-AzaGly-Leu-Arg(Me)-Trp-NH$_2$ ou un sel de ce composé, et un polymère d'acide lactique ou un sel d'un tel polymère.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200772997 A
- WO 0285399 A

- JP 2010144792 A

**Non-patent literature cited in the description**

- *Cancer Research,* 1989, vol. 49, 5935-5939
- *Journal of Clinical Oncology,* 1994, vol. 12, 213-225

- *British Journal of Cancer,* 1993, vol. 68, 314-318